# EUROPEAN PATENT APPLICATION

(11) **EP 2 762 494 A2**
(43) Date of publication of application: **06.08.2014**
(21) Application number: 13199443.6
(22) Date of filing: 23.12.2013
(51) Int. Cl.: C07K 16/18, G01N 33/68, G01N 33/577

(54) **Monoclonal antibodies against scrapie prion protein (PrPSc), and the use thereof**

(30) Priority: 25.12.2012 TW 101149718
(71) Applicant: National Yang-Ming University, Taipei City 112 (TW)
(72) Inventor: Chen, Yi-Ming Arthur, 112 Taipei (TW); Huang, Szu-Wei, 112 Taipei (TW); Lin, Ying-Yu, 112 Taipei (TW)
(74) Representative: Viering, Jentschura & Partner Patent- und Rechtsanwälte

(57) **Abstract**

The resent invention relates to monoclonal antibodies against PrP^{Sc} protein. The anti-PrP^{Sc} mAbs produced of this invention can specifically detect PrP^{Sc} protein from normal mouse brain homogenates without pre-treatment with protease K, which may be used in developing a diagnostic method and kit for prion disease with high specificity and sensitivity.

## Description

### Field of the Invention

The present invention relates to a monoclonal antibody specific to scrapie prion protein (PrP^{Sc}). In particular, the present invention relates to a monoclonal antibody specific to a peptide of prion protein having β sheet structure at C-terminus. The present invention further provides a method for detecting prion diseases by using the monoclonal antibody.

### Background of the Invention

Prion diseases, also known as transmissible spongiform encephalopathies (TSEs), are a group of rare degenerative brain disorders characterized by spongiform change in the brain of cattle, death of neuron cells, proliferation of astrocytes, and appearance of amyloid plaque in Histochemical staining. Symptoms of prion diseases commonly include personality changes, psychiatric problems such as depression, lack of coordination, and/or an unsteady gait. Patients also may experience involuntary jerking movements called myoclonus, unusual sensations, insomnia, confusion, or memory problems (Ludlam, C.A., Lancet 351:1289-1290, 1998; Soto, C., Nature reviews Microbiology 2:809-819, 2004).

Unlike other neurodegenerative disease, such as Alzheimer disease and Parkinson disease, prion diseases are transmissible, infectious, and fatal. Bovine Spongiform Encephalopathy (B S E) was first described in 1986 related to cattle feed containing meet and bones from carcasses of sheep being feed to bovines. Prion diseases can affect both humans and animals and are sometimes transmitted to humans by infected meat products. Prion diseases found in animals include Bovine Spongiform Encephalopathy (BSE), which affects cattle; and scrapie, which affects sheep and goats. Creutzfeldt-Jakob disease (CJD) is the most well-known of the human prion diseases. Other human TSEs include kuru, fatal familial insomnia (FFI), and Gerstmann-Straussler-Scheinker disease (GSS).

In 1986, the British had the first outbreak of the disease which caused a large number of cattle died; that is, later known as mad cow disease (Wells, GA, et al., The Veterinary record 121:419-420, 1987). Then, in next few years, some cases begun to appear in succession in other countries, resulting in a pandemic.

Studies have shown that BSE outbroke might be due to the bone powder as cattle feed additive, containing a lot of scrapie pathogen. It also implies that TSE might infect different species crossly, causing the diseases in other species. Human Creutzfeldt-Jakob disease was discovered in the early 1920s, as a very rare disease. The incidence is about 0.5∼1 per million population a case. The symptoms and pathology of the disease are similar to mad cow disease.

In the past few years, many countries have been convinced that BSE is a serious problem. Currently, in pathogen identification, the main sources of the samples are the brain tissue after "death". The mouse can only be artificially inoculated with the emulsion made from the brain tissues of the infectious cattle in order to confirm that the BSE pathogen is transmissible. However, such vaccine infection requires at least 150 days of infection in order to confirm whether the infection is established. Currently, the diagnostic methods recognized by WHO and OIE are based on postmortem examination of clinical disease, symptoms, microscopic pathology, immunohistochemistry staining and Western immunoblotting blotting, etc, as a general diagnosis of Prion. (Hardt, M., et al., Journal of comparative pathology 122:43-53, 2000; Ingrosso, L., et al., Trends in molecular medicine 8, 273-280, 2002).

Several TSE detection methods will be described as follows:
*Prionics-Check Western (Western blot, Prionics AG):* Simple automated one-step sample preparation followed by protease treatment. Detection occurs after the separation of the treated sample by denaturing polyacrylamide gel electrophoresis and transfer to a membrane using a PrP-specific antibody and an alkaline phosphatase-coupled secondary antibody detection system generating chemiluminescence. The presence of a PrP-immunoreactive signal with the additional two criteria of a reduced molecular weight (due to digestion of the N-terminus of PrP^{Sc}) and a typical 3-band pattern (due to different glycosylation forms of PrP) results in a TSE-positive diagnosis.
*Platelia test (ELISA, BioRad):* Sample preparation involving protease treatment followed by precipitation and a centrifugation step for enrichment of the analyte. Detection by sandwich ELISA using a colour-converting enzyme-coupled detection antibody. Cut-off setting with a grey zone that requires repetition of the assay.
*Conformation-dependent immunoassay, CDI (Inpro)* : The detection antibody recognizes a conformation-dependent epitope that, while always exposed in the non-infectious form (PrP^{C}), only becomes exposed in the infectious form of PrP (PrP^{Sc}) upon denaturation. Quantification of the binding events leads to a difference between the signals given by denatured PrP^{Sc} and native PrP^{Sc}, which is used as a diagnostic criterion.

The method described above has a potential problem due to its high detection limit (the CEA / Biorad detection currently has the lowest detection limit), leaving many cattle in the latent period of PrP^{Sc}. This causes false negative from the obtained chemical detection results. In addition, a similar situation may occur in human beings. Currently, the pre-symptom detection for the "Live" CJD patients is still under development. Thus, the same problem is that if vCJD patients are under an unknown condition for surgery, organ donation, blood transfusion and other medical practices, due to the characteristics of PrP^{Sc} for enduring high temperatures, fungicides, the contaminated surgical instruments are hardly to be cleaned thoroughly. Then it might occur iatrogenic CJD infection.

Furthermore, according to the characteristics of PrP^{Sc}, it usually requires a of proteolytic enzyme treatment after the specimen obtained. However, such treatment would cause the loss of PrP^{Sc}, escaping detection limits and obtaining another false negative result. To summarize, it is still in need to develop a novel detection method with high detection limits for the specimen in vivo which does not require the proteolytic enzyme treatment before occurring of the clinical symptoms.

Development of antibodies for specific binding to PrP^{Sc} structure has been important for the Prion disease research. The characterizations of the Prion protein are not like other infectious agents such as viruses, bacteria which can cause a strong immune response. The animals have the normal PrP protein expression (Prnp + / +). In the occurrence of the Prion disease, due to wide distribution of PrP^{C} on the surface of the normal B cells and T cells, the primary structure of PrP^{Sc} may be similar to PrP^{C}; thus, it causes immune tolerance of the B cell for no immune response (Bueler, H., et al., Cell 73, 1339-1347, 1993; Kascsak, R.J., et al., Journal of virology 61:3688, 1987; Prusiner, S.B., et al., The Journal of infectious diseases 167:602-613, 1993).

In the past few years, with different strategies, it has still been unable to develop a highly specific antibodies to identify PrP^{Sc}. In 1997, Korth team firstly used the recombinant bovine protein to produce 15B3. Although the antibody can immunoprecipitate PrP^{Sc} of cows, mice, and human beings but not PrP^{Sc}; in practical applications, it is relatively weak for the Prion protein binding. As a result, it cannot achieve the clinical diagnosis (Korth, C., et al., Nature 390:74, 1999). Therefore, it started to use full-length fragments (not including the N-terminal) of the immunogen to produce β sheeted recombinant protein for increasing the tyrosine residues. depending on tyrosine-tyrosine-arginine of different species repeating synthesis of peptide fragments (Paramithiotis, et al. 2003). Using scrapie-associated fibril (SAF) (Morel, N., et al., The Journal of biological chemistry 279:30143-30149, 2004) can also produce antibodies able to identify PrP^{Sc}; however, after experiments, it was found that they were still able to identify PrP^{C}. Up to now, there are many anti-PrP antibodies able to identify the various forms of PrP, but they are only for the purified native PrP^{Sc}, leaving only weak signals.

### Summary of the Invention

This invention is based on the unexpected discovery that mAbs generated from a mouse immunized with a recombinant β PrP can identify PrP^{Sc} from normal mouse brain homogenates without pre-treatment with protease K using both dot blot and Western blot assays.

Therefore, in one aspect, the present invention features a monoclonal antibody specific to scrapie prion protein (PrP^{Sc}), which binds to a peptide comprising the amino acid sequence of SEQ ID NO.1, composed of the amino acid residues 141-182 of prion protein.

In one embodiment of the invention, the monoclonal antibody may specifically recognize the β-sheet structure of PrP^{Sc}. In another embodiment of the invention, the peptide of prion protein is conformed to a β oligomer.

In some embodiments of the invention, the monoclonal antibody binds to a peptide comprising the amino acid sequence of SEQ ID NO.2, composed of the amino acid residues 141-152 of prion protein. In other embodiments of the invention, the monoclonal antibody binds to a peptide comprising the amino acid sequence of SEQ ID NO.3, composed of the amino acid residues 171-182 of prion protein.

In another aspect, this invention features a producing method of an antibody for specifically detecting scrapie prion protein (PrP^{Sc}), comprising preparing a recombinant prion protein with β-sheet structure (β PrP); immunizing a mouse with the recombinant PrP^{Sc} to obtain an antibody against the β PrP; and isolating and purifying the antibody. In one embodiment of the invention, the antibody is a monoclonal antibody. In another embodiment of the invention, the antibody is a polyclonal antibody.

In one embodiment of the invention, the β-sheet structure of the recombinant prion protein is refolded from the α-helical structures of PrP^{C}, and further polymerized into a β oligomer. In another embodiment of the invention, the recombinant prion protein comprises 4 amino acid mutations at Ser-132, Asn-181, Cys179, and Cys-214.

Further, the present invention provides a diagnostic kit for the specific detection of scrapie prion protein (PrP^{Sc}), characterized by comprising the monoclonal antibody of the invention.

In another aspect, the present invention relates to a method for the detection of prion diseases in animals or humans, comprising steps of: preparing a sample from brain tissue of the tested animal without protease treatment; and detecting the presence of infectious form of PrP (PrP^{Sc}) in the sample by using the monoclonal antibody of the invention. In some embodiments of the invention, the brain tissue sample is prepared as a brain tissue homogenate. In other embodiments of the invention, the brain tissue sample is prepared as a brain biopsy.

In one embodiment of the invention, the infectious form of PrP comprises a β-sheet structure. In another embodiment of the invention, the infectious form of PrP is conformed to a β oligomer.

Other features or advantages of the present invention will be apparent from the following drawings and detailed description of several embodiments, and also from the appending claims.

### Brief Description of the Drawings

Figure 1 shows the analysis of antibody secretion by hybridoma A2, A3, and 3B cultured in HAT selection using ELISA (Figure 1A) and Strip western blot (Figure 1B).
Figure 2 shows the isotyping of anti-mβPrP monoclonal antibodies mAb-A2, A3, and 3B.
Figure 3 is a diagram showing the recognition abilities of monoclonal antibodies mAb-A2 (Figure 3A), A3 (Figure 3B), and 3B (Figure 3C) for mouse βPrP and recombinant bovine PrP determined by dot plot analysis.
Figure 4 is a diagram showing the recognition abilities of monoclonal antibodies mAb-A2 (Figure 4A), A3 (Figure 4B), and 3B (Figure 4C) for mouse βPrP and αPrP determined by ELISA analysis.
Figure 5 shows the results of epitope sequencing analyzed by peptide scanning. Figures 5A-5C are ELISA analysis of 22 segments derived from the a.a. 93-230 of mouse PrP sequence coating on a 96-well plate. Figure 5D is a diagram showing the epitope sequence determined in the peptide scanning. Each of the corresponding monoclonal antibodies is listed below.
Figure 6 shows the difference in the recognition abilities of monoclonal antibodies mAb-A2, A3, 3B for normal mouse brain homogenates and infectious mouse brain homogenates by Western blot analysis. The samples include normal mouse brain homogenates, NBH (lane 3, 4, 7, 8, 11, 12, 14, 15) and infectious PrPSc (10%, 22L) mouse brain homogenates (lane 1, 2, 5, 6, 9, 10 and 13), with (+) or without (-) treatment of 50 µg/mL proteinase K.
Figure 7 is immunohistochemistry staining of cerebellum region from 22L PrP^{Sc} infected mouse brain slices and normal healthy mice brain slice (as normal control) with three PrP identifiable monoclonal antibodies -A2, A3, 3B (as listed in left). The Pre-immune group is used as Negative control. The magnification (100x and 200x) is showed under each column of slices.

### Detailed Description of the Invention

The characteristics and advantages of the present invention will be further illustrated and described in the following examples. The specific examples below are to be construed as merely illustrative, and not limitative of the remainder of the disclosure in any way whatsoever. Without further elaboration, it is believed that one skilled in the art can, based on the description herein, utilize the present invention to its fullest extent. All publications cited herein are hereby incorporated by reference in their entirety. Further, any mechanism proposed below does not in any way restrict the scope of the claimed invention.

### Embodiment 1: Production and characterization of monoclonal antibodies

### Preparing monoclonal antibody specific to scrapie prion protein (PrP^{Sc})

To obtain a recombinant prion protein with considered similar structure to scrapie prion protein, mouse prion protein was point mutated at Ser-132, Asn-181, Cys179, and Cys-214 for removing its disulfide bond and leading to the formation of β oligomers. The recombinant mutant mouse prion protein (so called as mouse β PrP, mβPrP) generated from *E. coli* was analyzed by Electron paramagnetic resonance (EPR) and Circular dichroism, and found that its α-helix structure at C-terminal may turn to β-sheet and further be polymerized into a β oligomer at the condition of low salt concentration, pH 7, at room temperature. The mouse β PrP, which is considered to have a structure similar to PrP^{Sc}, was used as antigen for immunizing mice to generate antibodies.

Each BABL/c mouse was injected peritoneally with 50 µg of mouse β PrP in 1X PBS (250 µl), emulsified with 250 µl of complete Freund's adjuvant. After 2 weeks of the initial injection, subsequent inoculations were given peritoneally at 2-week intervals with 50 µg of mouse β PrP in 1X PBS (250 µl), emulsified with 250 µl of incomplete Freund's adjuvant. Blood samples were taken after one week of each boosting injection, and the production of anti-β PrP antibody was detected in the isolated serum samples by ELISA. After five boosting injection, the mice producing anti-β PrP antibodies were determined and sacrificed for the preparation of hybridoma cell lines.

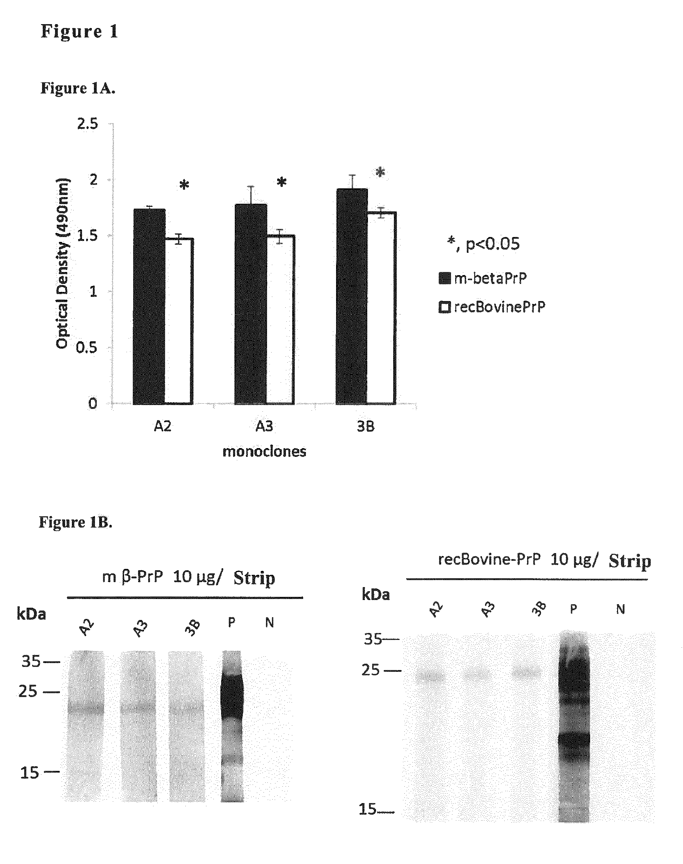

After immunization with mβPrP recombinant proteins, it will promote B cells in the spleen to produce specific antibodies. Fusion of cancerous cells can be cultured in vitro into strains of specific antibodies. In this study, spleen cells of the immunized mice (BABL/c mice) were fused with the myeloma cell line NS-1. Then the cells were cultured in HAT growth medium, dispensed into six 96-well culture plates, and incubated for 7 to 14 days. By using three kinds of limiting dilution (including rapid cell dilution method, slow cell dilution method and cell counting method) to get monoclonal antibodies produced by a single cell clone, and the specificity of each monoclonal antibody is confirmed by enzyme immunoassay and Western blotting. Finally, three mβPrP monoclonal antibodies clones A2, A3 and 3B were obtained, as shown in Figure 1.

Next, the monoclonal antibodies were implanted into abdomen of mice to obtain high yield of monoclonal antibodies produced in the ascitic fluid. The HAT medium in monoclonal antibody cell culture is toxic for mice; thus, the selected monoclonal antibodies must be cultured in RPMI cell culture medium before implanted into mice body for obtaining high level of monoclonal antibodies. The produced monoclonal antibodies in ascetic fluid are further purified by chromatography. 0.5ml protein G plus protein A agarose is filled into a column, and washed with 1ml 1X PBS. 6ml of a mixture of equal amounts of ascitic fluid and 1X PBS is loaded to the column, with flowing through the column for 3 to 5 times repeatly. 5 ml of 1X PBS is added to wash the agarose. 10 ml of 0.5 M NaOAC (pH = 3) + 0.01% sodium azide was then added to the column, and the first batch of purified antibodies was obtained. The addition of the sodium azide solution was repeated for 3-5 times. 2.5 ml of 2 M Tris base + 0.01% sodium azide was added to balance the pH value of the solution of purified antibodies. 5ml of 10mM glycine (pH=3) + 0.01% was then added to the column, and the second batch of purified antibodies was obtained. The addition of the sodium azide solution was repeated for 3-5 times. 1.25 ml of 2M Tris base + 0.01% sodium azide was added to balance the pH value of the solution of purified antibodies. The first and second batches of purified antibodies were combined and applied to a concentration tube. The solution of purified monoclonal antibody was concentrated to 1 ml after centrifugation. The concentration of monoclonal antibody was determined and aliquot to 100 ug in each tube for phage display technique, or the remains were stored at -20°C/-80°C.

### Isotyping of anti-mβPrP monoclonal antibodies mAb-A2, A3, and 3B

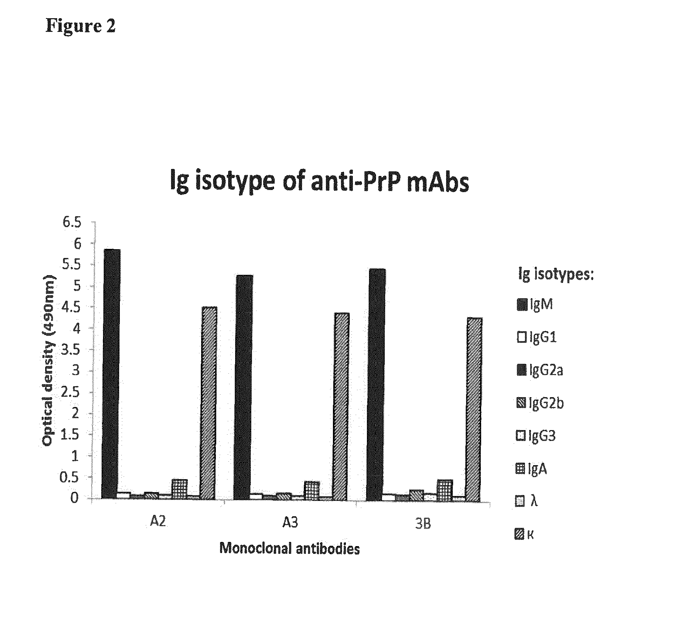

A 96-well ELISA plate was coated with 2.5 ug/ml of immunoglobulin in each well. The supernatants obtained from the culture of hybridoma cell lines A2, A3, and 3B were used as primary antibody in the assay. Then, different immunoglobulins (including HRP-conjugated anti-IgA, IgM, IgG1, IgG2a, IgG2b and IgG3 antibodies for heavy chain identification; and HRP-conjugated anti-κ chain and anti-λ chain antibodies for light chain identification) were used as secondary antibody. The isotypies of heavy and light chains of the monoclonal antibodies A2, A3, and 3B are determined at wavelength of 490 nm. The results show that the heavy chain and the light chain of the monoclonal antibodies A2, A3 and 3B obtained in the present invention are IgM and κ chain, respectively (see, Figure 2).

### Using dot plot method to compare differences in identification of monoclonal antibodies A2, A3 and 3B to different amounts of mβPrP and recombinant bovine PrP

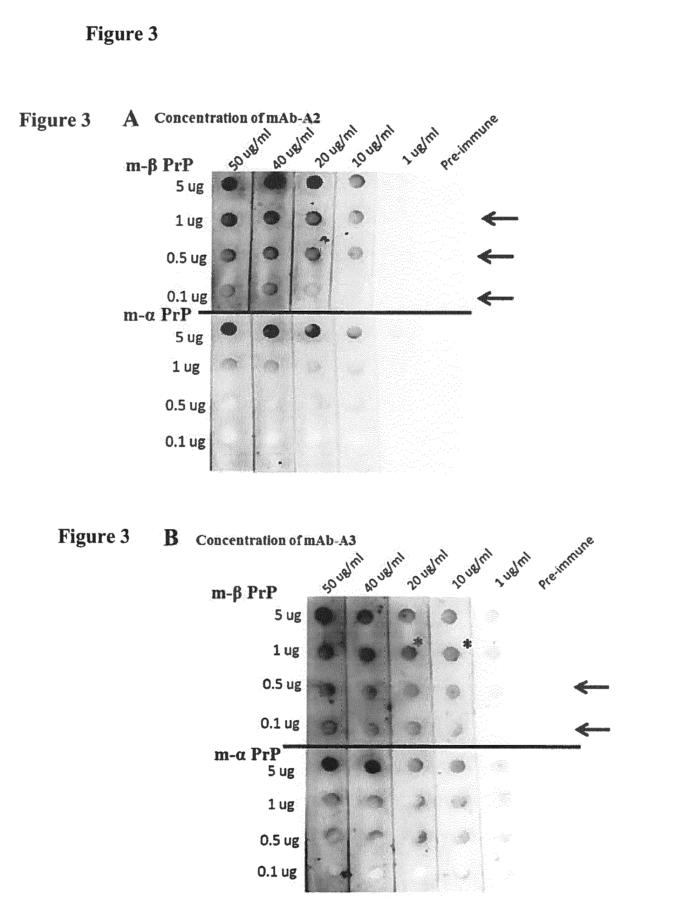

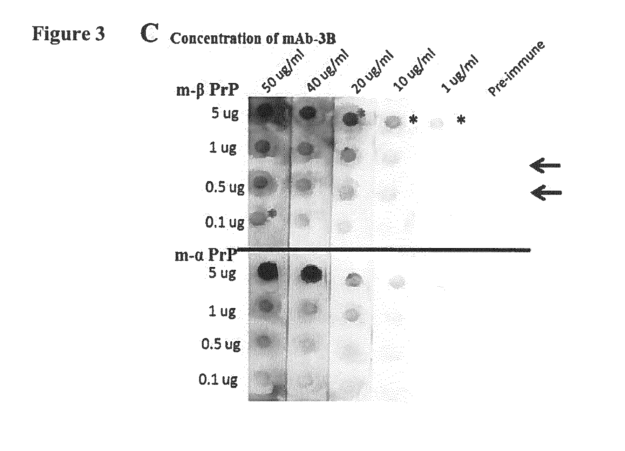

The PrP proteins of the same species will exhibit different types of three-dimensional structure in native state by using Dot blot analyze, wherein the mαPrP exists as a monomer and the mβPrP exists as an oligomer. 5µg, 1µg, 0.5 µg, and 0.1µg of mβPrP and mαPrP were doted on a PVDF blotting membrane. Then, 5% milk was added for blocking. Monoclonal antibodies A2, A3, 3B at different dilution were used as the primary antibodies. The mouse pre-immune serum is used as the negative control. Results are showed in Figure 3.

Figure 3A shows that A2 monoclonal antibody at 10 µg/ml to 50 µg/ml has better identification limit for mβPrP; even 0.1 µg of mβPrP protein is detectable. However, 5 mαPrP can be detected up to 1µg. A2 monoclonal antibody below 1 µg/ml has weak signal for 10µg mαPrP, none for mβPrP. Figure 3B shows that the identification signal of A3 monoclonal antibody at 10µg/ml to 50 µg/ml for mβPrP is better that that of mαPrP, especially more significant at asterisk. However, under 1µg/ml of antibody, it makes no difference for the recognition to the two proteins.

Figure 3 C shows that the identification signal of monoclonal antibody 3B at 1 µg/ml to 20 µg/ml for 5 µg mβPrP is better than that of mαPrP. In addition, monoclonal antibody 3B exhibits better identification for 0.1 µg mβPrP than that of mαPrP at 50 µg/ml. Others do not make any difference. To summarize, all the three monoclonal antibodies mAb A2, A3, 3B can indentify mαPrP or mβPrP, and showed a better detection limit for mβPrP than for mαPrP at a concentration of 10 µg/ml to 50 µg/ml.

### ELISA analysis of anti-PrP monoclonal antibodies A2, A3, 3B for identification differences of mβPrP and αPrP

ELISA analysis was also conducted for showing the recognition of each monoclonal antibody to different proteins presented as certain values. ELISA plates are coated with 200 ng/well of the mouse βPrP, αPrP proteins. Then, mAbs A2, A3, 3B monoclonal antibodies at different dilution are used as the primary antibody, and the anti-mouse IgM antibody (1:4000) is used as secondary antibody. The OPD Color development is preceded after the binding reaction of the antibodies.

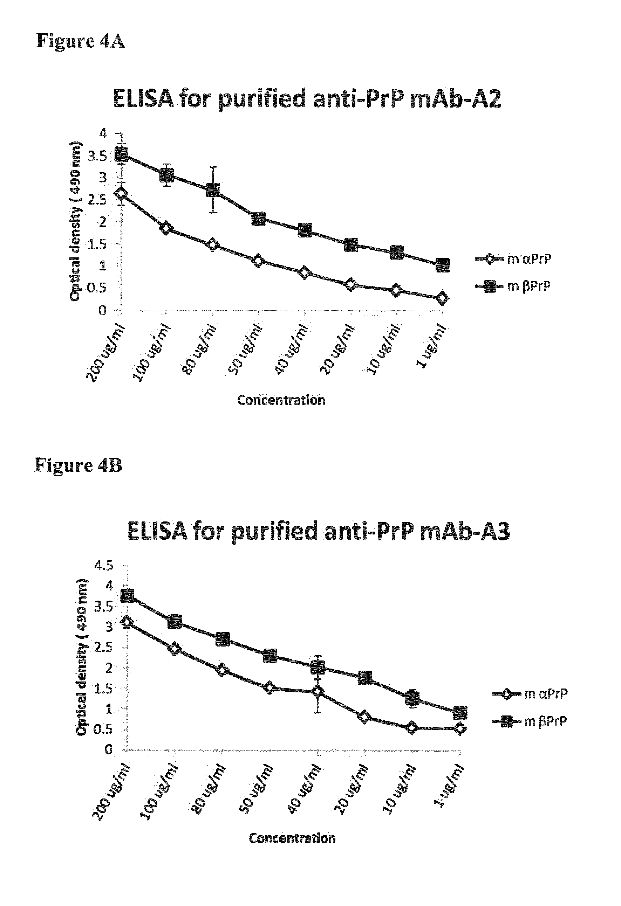

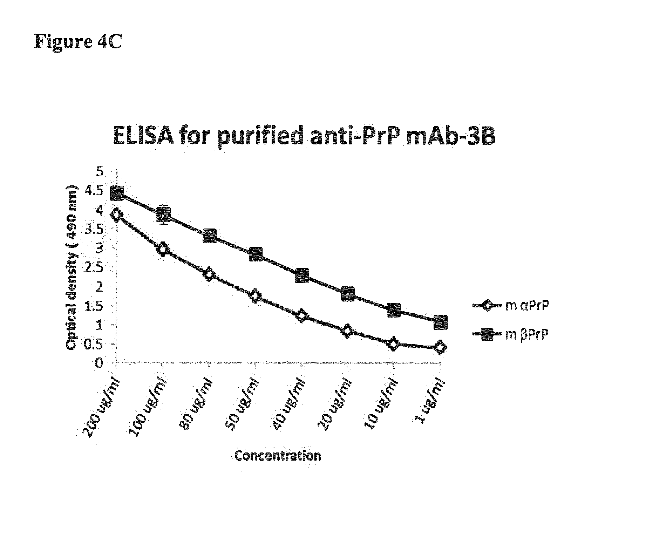

The results showed that monoclonal antibodies A2, A3 and 3B have better recognition for mβPrP at 1 µg/ml to 200 µg/ml, as compared to those for mαPrP (see Figure 4). The overall recognition results, including Figure 1, show that the monoclonal antibody of the present invention can recognize the PrP proteins from another species (bovine) as present in native form, and exhibit different affinities for the monomer and oligomer.

### Embodiment 2: Analysis of epitopes on prion protein recognized by the anti-mβPrP monoclonal antibodies

### Peptide scanning

The a.a. 93-230 fragment of mPrP sequence is divided into 22 segments, each has 12 amino acids with six amino acid overlapping. A 96-well ELISA plate is coated with the peptide fragments for ELISA assay. The purified A2, A3, and 3B monoclonal antibodies are used as primary antibodies, and the HRP-conjugated anti-mouse IgM polyclonal antibody is used as secondary antibody.

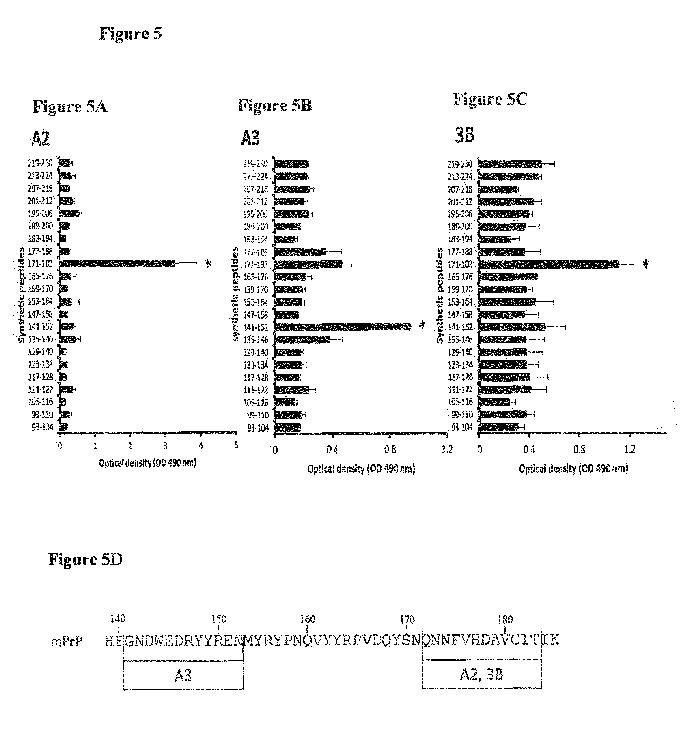

The results of peptide scanning analysis are shown in Figure 5. It is found that the epitope of monoclonal antibody A3 is located in the a.a. 141-152 GNDWEDRYYREN (SEQ ID NO. 2) fragment of mPrP sequence, and the epitopes of monoclonal antibodies A2 and 3B are in the same fragment, a.a. 171-182 QNNFVHDAVCIT (SEQ ID NO. 3).

### Phage display technique

Phage colonies are randomly selected from the phage clones those had been screened in four rounds and their DNA are extracted for sequencing. Sequences from selected phage clones are listed as follow. The parentheses showed after each sequence represents (number of occurrence/total selected sequence number). Bold means a sequence appears in both cross-compared sequences. The possible epitope sequence is summarized in the bottom line of tables. The underline represents the sequence appears in the back comparison.

| **Antibody: mAb-A2** | |
|---|---|
| Sequence from phage clons | **PVSAV**RP (5/10) |
| | TA**PVS**PI/S(2/10) |
| | PGQD**AVM** (1/10) |
| | GPRDPRD (1/10) |
| | TAAG-S- (1/10) |
| Possible epitope | **164-** R**PVD**QYSNQNNFVHD**AV**CIT-**182** |

| **Antibody: mAb-A3** | |
|---|---|
| Sequence from phage clons | RVNTK L/P I (11/20) |
| | LKTRVIS (5/20) |
| | TPARHIY (1/20) |
| | AYPSAHR (1/20) |
| | - RLVLF- (1/20) |
| Possible epitope | NA |

| **Antibody: mAb-3B** | |
|---|---|
| Sequence from phage clons | DI**P**Y**Q**Q F/**S** (10/20) |
| | **RPK**xxxx (1/20) |
| | ASNRPYV (7/20) |
| | ITLSGGI (1/20) |
| | TPPRHIY (1/20) |
| Possible epitope | 23-MKK**RPK**PGGWNTGGSRY**P**G**Q**G**S**P-45 |

To summarize, 5 phage sequences were totally obtained in the mAb-A2 selection and the peptide sequence 163-RPVDQYSNQNNFVHDAVCIT-182 (SEQ ID NO. 4) was found to be a possible epitope in the alignment of repeat sequences appeared in the cross-comparison and the mouse PrP sequence. There is no repeat sequence found in the 5 sequences obtained in mAb-A3 selection. The possible epitope of mAb-3B was obtained in the alignment of 5 sequences, and found to be at the N-terminal 23-MKKRPKPGGWNTGGSRYPGQGSP-45 (SEQ ID NO. 5).

Therefore, epitope of mAb-A3 is at the sequence of 141-152 GNDWEDRYYR EN (SEQ ID NO. 2); and epitopes of A2, 3B are in the same position, at the sequence of 171-182 QNNFVHDAVC IT (SEQ ID NO. 3). Three epitopes of these monoclonal antibodies are highly conserved in different species.

### Embodiment 3: Recognition of anti-PrP^{Sc} monoclonal antibodies for brain tissues in normal and infectious mice

### Western blotting

In this experiment, western blot analysis was used to identify the difference between mAb-A2, A3, 3B to normal mouse brain homogenates and infectious mouse brain homogenates. The sample is taken from normal mouse brain homogenate, NBH (lane 3, 4, 7, 8, 11, 12, 14, 15); and infectious PrPSc (10%, 22L) mouse brain homogenate (lane 1, 2, 5, 6, 9, 10 and 13), with (+) or without (-) treatment of 50 µg/mL proteinase K at 37°C for 1 hour. 5 mM PMSF was added for 10 min to terminate the reaction. The samples were subjected to SDS-PAGE and transferred onto a PVDF membrane for Western blotting. The mAb-A2, A3, 3B, SAF32 were used as primary antibodies; and the HRP-conjugated anti-mouse IgM, IgG antibodies were used as secondary antibodies (1:10000); then followed with ECL development.

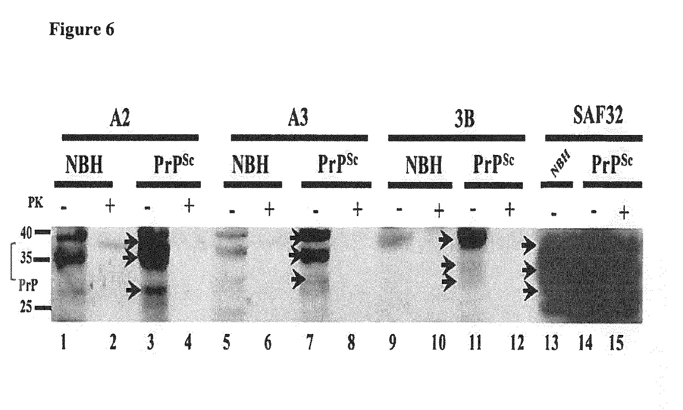

From Western blot results, it can be found that, in the absence of proteinase K treatment, the monoclonal antibodies of the present invention are able to recognize PrP^{Sc}**,** and identify different patterns of glycosylation, with stronger recognition signal than that from the normal mouse brain (see Figure 6). From the above results, monoclonal antibodies of the present invention can be applied to the development of diagnostic method and kits for specific recognition to PrP^{Sc} used before the clinical symptoms occur in vivo, without need of proteinase reaction of the specimen. It is able to directly identify the presence of PrP^{Sc}, effectively saving labor and time of sample processing and detection.

### Immunohistochemistry

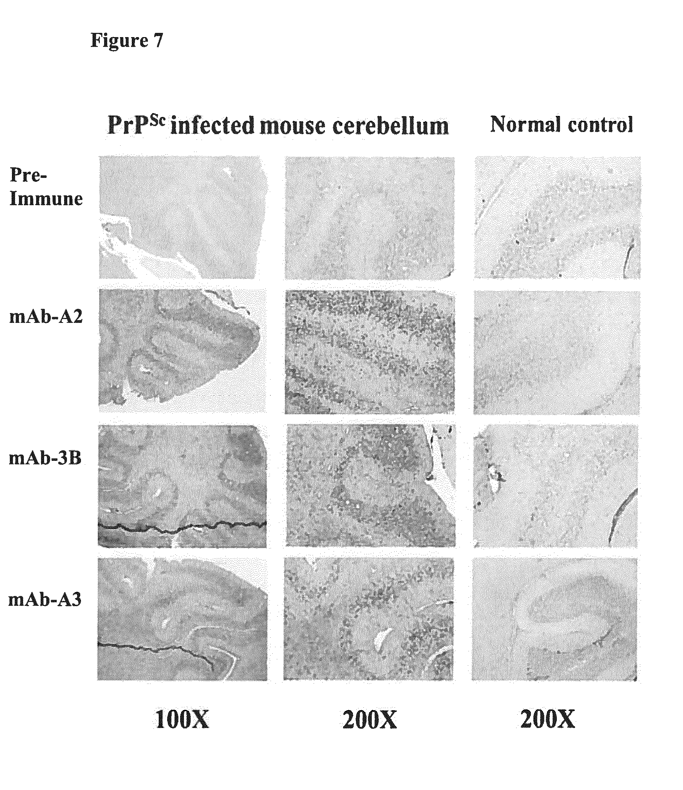

Immunohistochemistry staining of 22L PrP^{Sc} infected mouse brain slices and normal healthy mice brain slice (as normal control) were performed by using three PrP identifiable monoclonal antibodies -A2, A3, 3B obtained in the present invention to detect the PrP^{Sc} expression in mouse brain tissues. Slices of the sample taken from normal mouse brain (as normal control) and infectious mouse brain were stained and observed under a microscope and photographed (with magnification of 100x and 200x), mainly around cerebellum region. The pre-immune serum is used as negative control. The results are shown in Figure 7.

From the results of immunohistochemistry, it can be found that monoclonal antibody A2 shows plaque signals, mainly in the gray matter of the cerebellum granular layer, near the molecular layer of nerve cells. The signal of monoclonal antibody 3B also distributed in the granular layer of the cerebellum and the molecular layer in point-like manner. While the immunohistochemistry signal of monoclonal antibody A3 is diffuse in the small white matter. However, there were no significant signals observed in normal mouse brain tissue when stained with these antibodies.

Since epitopes of the monoclonal antibody obtained in the present invention are highly conserved in species, it can be used to detect various species of infectious prion proteins, as well as for detecting prion diseases, including BSE, scrapie, the different types of human Creutzfeldt-Jakob disease CJD (including sporadic, variant, atrogenic, familial, etc.). Moreover, the monoclonal antibody of the present invention is able to use in the clinical detection of PrP^{Sc}, for example used in the immunohistochemistry staining and Capture EIA techniques for blood and cerebrospinal fluid specimens. The monoclonal antibody of the present invention can also combine with Surface Plasmon Resonance (SPR) technology to further enhance the detection limit and sensitivity.

### Other Embodiments

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the spirit and scope thereof, can make various changes and modifications of the invention to adapt it to various usages and conditions. Thus, other embodiments are also within the claims.

## Claims

1. A monoclonal antibody specific to scrapie prion protein (PrP^{Sc}), which binds to a peptide comprising the amino acid sequence of SEQ ID NO.1, composed of the amino acid residues 141-182 of prion protein.

2. The monoclonal antibody of claim 1, wherein the monoclonal antibody specifically recognizes the β-sheet structure of PrP^{Sc}.

3. The monoclonal antibody of claim 1 or 2, wherein the monoclonal antibody specifically recognizes the prion protein which is conformed to a β oligomer.

4. The monoclonal antibody of claim 1, wherein the monoclonal antibody binds to a peptide comprising the amino acid sequence of SEQ ID NO.2, composed of the amino acid residues 141-152 of prion protein.

5. The monoclonal antibody of claim 1, wherein the monoclonal antibody binds to a peptide comprising the amino acid sequence of SEQ ID NO.3, composed of the amino acid residues 171-182 of prion protein.

6. A producing method of an antibody for specifically detecting scrapie prion protein (PrP^{Sc}), comprising:
preparing a recombinant prion protein with β-sheet structure (β PrP);
immunizing a mouse with the recombinant PrP^{Sc} to obtain an antibody against the β PrP; and
isolating and purifying the antibody.

7. The method of claim 6, wherein the antibody includes a monoclonal antibody and a polyclonal antibody.

8. The method of claim 6, wherein the β-sheet structure of the recombinant prion protein is refolded from the α-helical structures of PrP^{C}, and further polymerized into a β oligomer.

9. The method of claim 6, wherein the recombinant prion protein comprises 4 amino acid mutations at Ser-132, Asn-181, Cys179 and Cys-214.

10. A diagnostic kit for specifically detecting scrapie prion protein (PrP^{Sc}), which is **characterized by** comprising the monoclonal antibody of claim 1.

11. A method for the detection of prion diseases in animals or humans, comprising steps of:
(a) preparing a sample from brain tissue of the tested animal without protease treatment; and
(b) detecting the presence of infectious form of PrP (PrP^{Sc}) in the sample by a immunoassay using the monoclonal antibody of claim 1.

12. The method of claim 11, wherein the brain tissue sample is prepared as a brain tissue homogenate.

13. The method of claim 12, wherein the immunoassay is Western blot assay.

14. The method of claim 11, wherein the brain tissue sample is prepared as a brain tissue slice.

15. The method of claim 14, wherein the immunoassay is immunohistochemistry assay.

16. The method of claim 11, wherein the infectious form of PrP comprises a β-sheet structure.

17. The method of claim 16, wherein the infectious form of PrP is conformed to a β oligomer.


SEQUENCE LISTING

<110> National Yang-Ming University

<120> Monoclonal Antibodies against Scrapie Prion Protein (PrPSc), and the
Use Thereof

<130> P 50874

<140>
<141> 2013-12-24

<150> TW 101149718
<151> 2012-12-25

<160> 5

<170> FastSEQ for Windows Version 4.0

<210> 1
<211> 42
<212> PRT
<213> Artificial sequence

<220>
<223> mPrP141-182

<400> 1
Gly Asn Asp Trp Glu Asp Arg Tyr Tyr Arg Glu Asn Met Tyr Arg Tyr Pro
1               5                   10                  15
Asn Gln Val Tyr Tyr Arg Pro Val Asp Gln Tyr Ser Asn Gln Asn Asn Phe
        20                  25                  30
Val His Asp Ala Val Cys Ile Thr
35                  40

<210> 2
<211> 12
<212> PRT
<213> Artificial sequence

<220>
<223> mPrP141-152

<400> 2
Gly Asn Asp Trp Glu Asp Arg Tyr Tyr Arg Glu Asn
1               5                   10

<210> 3
<211> 12
<212> PRT
<213> Artificial sequence

<220>
<223> mPrP171-182

<400> 3
Gln Asn Asn Phe Val His Asp Ala Val Cys Ile Thr
1               5                   10

<210> 4
<211> 20
<212> PRT

<213> Artificial sequence

<220>
<223> mPrP163-182

<400> 4
Arg Pro Val Asp Gln Tyr Ser Asn Gln Asn Asn Phe Val His Asp Ala Val
1               5                   10                  15
Cys Ile Thr
        20

<210> 5
<211> 23
<212> PRT
<213> Artificial sequence

<220>
<223> mPrP23-45

<400> 5
Met Lys Lys Arg Pro Lys Pro Gly Gly Trp Asn Thr Gly Gly Ser Arg Tyr
1               5                   10                  15
Pro Gly Gln Gly Ser Pro
        20